# FASCICULE DE BREVET EUROPEEN

(11) **EP 2 906 593 B1**
(45) Date de publication et mention de la délivrance du brevet: **11.03.2020**
(21) Numéro de dépôt: 13808227.6
(22) Date de dépôt: 09.10.2013
(51) Int. Cl.: C07K 14/47

(54) **PROCEDE DE PREPARATION DE PROTEINE C1Q RECOMBINANTE**
VERFAHREN ZUR HERSTELLUNG VON REKOMBINANTEM C1Q-PROTEIN
METHOD FOR PREPARING C1Q RECOMBINANT PROTEIN

(30) Priorité: 09.10.2012 FR 1259635
(43) Date de publication de la demande: 19.08.2015
(73) Titulaire: Commissariat à l'Énergie Atomique et aux Énergies Alternatives, 75015 Paris (FR); INSTITUT NATIONAL DE LA SANTE ET DE LA RECHERCHE MEDICALE (INSERM), 75013 Paris (FR)
(72) Inventeur: THIELENS, Nicole, F-38140 St Blaise du Buis (FR); BALLY, Isabelle, F-38120 Fontanil Cornillon (FR)
(74) Mandataire: Gevers & Orès
(86) Numéro de dépôt international: PCT/IB2013/059236
(87) Numéro de publication internationale: WO 2014/057437

(56) Documents cités:
- "C1QC (NM_172369) Purified Human Protein", Origene, product catalog, cat. no. TP303832, 16 mai 2012 (2012-05-16), pages 1-1, XP002697854, Extrait de l'Internet: URL:http://www.origene.com/protein/product .aspx?product=TP303832 [extrait le 2013-05-29]
- "C1QC Over-expression Lysate Product", Origene, product catalog, cat. no. LY403541, 4 mai 2011 (2011-05-04), page 1, XP002697855, Extrait de l'Internet: URL:http://www.origene.com/lysate/LY403541 .aspx [extrait le 2013-05-29]
- "C1QC (NM_172369) Human cDNA ORF Clone", Origene, product catalog, cat. no.RC203832, 4 mai 2011 (2011-05-04), page 1, XP002697856, Extrait de l'Internet: URL:http://www.origene.com/Human_ORF/RC203 832.aspx [extrait le 2013-05-29] -& "ORF nucleotide sequence for C1QC human cDNA clone, cat. no. RC203832", , 4 mai 2011 (2011-05-04), XP002697857, Extrait de l'Internet: URL:http://www.origene.com/orf_clone/searc h/orf_nt.aspx?est_name=MK6183_E05&sku=RC20 3832&type=Human [extrait le 2013-05-29] -& "Protein sequence of C1QC human cDNA clone, cat. no. RC203832", , 4 mai 2011 (2011-05-04), XP002697858, Extrait de l'Internet: URL:http://www.origene.com/orf_clone/searc h/orf_aa.aspx?est_name=MK6183_E05&sku=RC20 3832&type=Human [extrait le 2013-05-29]
- J. ZHANG ET AL: "Influenza A virus M1 blocks the classical complement pathway through interacting with C1qA", JOURNAL OF GENERAL VIROLOGY, vol. 90, no. 11, 1 novembre 2009 (2009-11-01), pages 2751-2758, XP055064462, ISSN: 0022-1317, DOI: 10.1099/vir.0.014316-0
- R. LI ET AL: "Tumor Necrosis Factor Death Receptor Signaling Cascade Is Required for Amyloid- Protein-Induced Neuron Death", JOURNAL OF NEUROSCIENCE, vol. 24, no. 7, 18 février 2004 (2004-02-18), pages 1760-1771, XP055064470, ISSN: 0270-6474, DOI: 10.1523/JNEUROSCI.4580-03.2004
- GABORIAUD CHRISTINE ET AL: "The crystal structure of the globular head of complement protein C1q provides a basis for its versatile recognition properties", JOURNAL OF BIOLOGICAL CHEMISTRY, AMERICAN SOCIETY FOR BIOCHEMISTRY AND MOLECULAR BIOLOGY, US, vol. 278, no. 47, 21 novembre 2003 (2003-11-21), pages 46974-46982, XP002597310, ISSN: 0021-9258, DOI: 10.1074/JBC.M307764200 [extrait le 2003-09-05] cité dans la demande

### LISTAGE DES SEQUENCES

<110> Commissariat à l'énergie atomique et aux énergies alternatives (CEA)
<120> PROCEDE DE PREPARATION DE PROTEINE C1Q RECOMBINANTE
<130> F263-529
<160> 24
<170> PatentIn version 3.5
<210> 1
   <211> 223
   <212> PRT
   <213> Homo sapiens
<400> 1
   Glu Asp Leu Cys Arg Ala Pro Asp Gly Lys Lys Gly Glu Ala Gly Arg
   1               5                   10                  15
   Pro Gly Arg Arg Gly Arg Pro Gly Leu Lys Gly Glu Gln Gly Glu Pro
               20                  25                  30
   Gly Ala Pro Gly Ile Arg Thr Gly Ile Gln Gly Leu Lys Gly Asp Gln
           35                  40                  45
   Gly Glu Pro Gly Pro Ser Gly Asn Pro Gly Lys Val Gly Tyr Pro Gly
       50                  55                  60
   Pro Ser Gly Pro Leu Gly Ala Arg Gly Ile Pro Gly Ile Lys Gly Thr
   65                  70                  75                  80
   Lys Gly Ser Pro Gly Asn Ile Lys Asp Gln Pro Arg Pro Ala Phe Ser
                   85                  90                  95
   Ala Ile Arg Arg Asn Pro Pro Met Gly Gly Asn Val Val Ile Phe Asp
               100                 105                 110
   Thr Val Ile Thr Asn Gln Glu Glu Pro Tyr Gln Asn His Ser Gly Arg
           115                 120                 125
   Phe Val Cys Thr Val Pro Gly Tyr Tyr Tyr Phe Thr Phe Gln Val Leu
       130                 135                 140
   Ser Gln Trp Glu Ile Cys Leu Ser Ile Val Ser Ser Ser Arg Gly Gln
   145                 150                 155                 160
   Val Arg Arg Ser Leu Gly Phe Cys Asp Thr Thr Asn Lys Gly Leu Phe
                   165                 170                 175
   Gln Val Val Ser Gly Gly Met Val Leu Gln Leu Gln Gln Gly Asp Gln
               180                 185                 190
   Val Trp Val Glu Lys Asp Pro Lys Lys Gly His Ile Tyr Gln Gly Ser
           195                 200                 205
   Glu Ala Asp Ser Val Phe Ser Gly Phe Leu Ile Phe Pro Ser Ala
       210                 215                 220
<210> 2
   <211> 245
   <212> PRT
   <213> Homo sapiens
<400> 2
<210> 3
   <211> 228
   <212> PRT
   <213> Homo sapiens
<400> 3
   Gln Ala Gln Leu Ser Cys Thr Gly Pro Pro Ala Ile Pro Gly Ile Pro
   1               5                   10                  15
   Gly Ile Pro Gly Thr Pro Gly Pro Asp Gly Gln Pro Gly Thr Pro Gly
               20                  25                  30
   Ile Lys Gly Glu Lys Gly Leu Pro Gly Leu Ala Gly Asp His Gly Glu
           35                  40                  45
   Phe Gly Glu Lys Gly Asp Pro Gly Ile Pro Gly Asn Pro Gly Lys Val
       50                  55                  60
   Gly Pro Lys Gly Pro Met Gly Pro Lys Gly Gly Pro Gly Ala Pro Gly
   65                  70                  75                  80
   Ala Pro Gly Pro Lys Gly Glu Ser Gly Asp Tyr Lys Ala Thr Gln Lys
                   85                  90                  95
   Ile Ala Phe Ser Ala Thr Arg Thr Ile Asn Val Pro Leu Arg Arg Asp
               100                 105                 110
   Gln Thr Ile Arg Phe Asp His Val Ile Thr Asn Met Asn Asn Asn Tyr
           115                 120                 125
   Glu Pro Arg Ser Gly Lys Phe Thr Cys Lys Val Pro Gly Leu Tyr Tyr
       130                 135                 140
   Phe Thr Tyr His Ala Ser Ser Arg Gly Asn Leu Cys Val Asn Leu Met
   145                 150                 155                 160
   Arg Gly Arg Glu Arg Ala Gln Lys Val Val Thr Phe Cys Asp Tyr Ala
                   165                 170                 175
   Tyr Asn Thr Phe Gln Val Thr Thr Gly Gly Met Val Leu Lys Leu Glu
               180                 185                 190
   Gln Gly Glu Asn Val Phe Leu Gln Ala Thr Asp Lys Asn Ser Leu Leu
           195                 200                 205
   Gly Met Glu Gly Ala Asn Ser Ile Phe Ser Gly Phe Leu Leu Phe Pro
       210                 215                 220
   Asp Met Glu Ala
   225
<210> 4
   <211> 251
   <212> PRT
   <213> Homo sapiens
<400> 4
   Met Lys Ile Pro Trp Gly Ser Ile Pro Val Leu Met Leu Leu Leu Leu
   1               5                   10                  15
   Leu Gly Leu Ile Asp Ile Ser Gln Ala Gln Leu Ser Cys Thr Gly Pro
               20                  25                  30
   Pro Ala Ile Pro Gly Ile Pro Gly Ile Pro Gly Thr Pro Gly Pro Asp
           35                  40                  45
   Gly Gln Pro Gly Thr Pro Gly Ile Lys Gly Glu Lys Gly Leu Pro Gly
       50                  55                  60
   Leu Ala Gly Asp His Gly Glu Phe Gly Glu Lys Gly Asp Pro Gly Ile
   65                  70                  75                  80
   Pro Gly Asn Pro Gly Lys Val Gly Pro Lys Gly Pro Met Gly Pro Lys
                   85                  90                  95
   Gly Gly Pro Gly Ala Pro Gly Ala Pro Gly Pro Lys Gly Glu Ser Gly
               100                 105                 110
   Asp Tyr Lys Ala Thr Gln Lys Ile Ala Phe Ser Ala Thr Arg Thr Ile
           115                 120                 125
   Asn Val Pro Leu Arg Arg Asp Gln Thr Ile Arg Phe Asp His Val Ile
       130                 135                 140
   Thr Asn Met Asn Asn Asn Tyr Glu Pro Arg Ser Gly Lys Phe Thr Cys
   145                 150                 155                 160
   Lys Val Pro Gly Leu Tyr Tyr Phe Thr Tyr His Ala Ser Ser Arg Gly
                   165                 170                 175
   Asn Leu Cys Val Asn Leu Met Arg Gly Arg Glu Arg Ala Gln Lys Val
               180                 185                 190
   Val Thr Phe Cys Asp Tyr Ala Tyr Asn Thr Phe Gln Val Thr Thr Gly
           195                 200                 205
   Gly Met Val Leu Lys Leu Glu Gln Gly Glu Asn Val Phe Leu Gln Ala
       210                 215                 220
   Thr Asp Lys Asn Ser Leu Leu Gly Met Glu Gly Ala Asn Ser Ile Phe
   225                 230                 235                 240
   Ser Gly Phe Leu Leu Phe Pro Asp Met Glu Ala
                   245                 250
<210> 5
   <211> 217
   <212> PRT
   <213> Homo sapiens
<400> 5
   Asn Thr Gly Cys Tyr Gly Ile Pro Gly Met Pro Gly Leu Pro Gly Ala
   1               5                   10                  15
   Pro Gly Lys Asp Gly Tyr Asp Gly Leu Pro Gly Pro Lys Gly Glu Pro
               20                  25                  30
   Gly Ile Pro Ala Ile Pro Gly Ile Arg Gly Pro Lys Gly Gln Lys Gly
           35                  40                  45
   Glu Pro Gly Leu Pro Gly His Pro Gly Lys Asn Gly Pro Met Gly Pro
       50                  55                  60
   Pro Gly Met Pro Gly Val Pro Gly Pro Met Gly Ile Pro Gly Glu Pro
   65                  70                  75                  80
   Gly Glu Glu Gly Arg Tyr Lys Gln Lys Phe Gln Ser Val Phe Thr Val
                   85                  90                  95
   Thr Arg Gln Thr His Gln Pro Pro Ala Pro Asn Ser Leu Ile Arg Phe
               100                 105                 110
   Asn Ala Val Leu Thr Asn Pro Gln Gly Asp Tyr Asp Thr Ser Thr Gly
           115                 120                 125
   Lys Phe Thr Cys Lys Val Pro Gly Leu Tyr Tyr Phe Val Tyr His Ala
       130                 135                 140
   Ser His Thr Ala Asn Leu Cys Val Leu Leu Tyr Arg Ser Gly Val Lys
   145                 150                 155                 160
   Val Val Thr Phe Cys Gly His Thr Ser Lys Thr Asn Gln Val Asn Ser
                   165                 170                 175
   Gly Gly Val Leu Leu Arg Leu Gln Val Gly Glu Glu Val Trp Leu Ala
               180                 185                 190
   Val Asn Asp Tyr Tyr Asp Met Val Gly Ile Gln Gly Ser Asp Ser Val
           195                 200                 205
   Phe Ser Gly Phe Leu Leu Phe Pro Asp
       210                 215
<210> 6
   <211> 245
   <212> PRT
   <213> Homo sapiens
<400> 6
   Met Asp Val Gly Pro Ser Ser Leu Pro His Leu Gly Leu Lys Leu Leu
   1               5                   10                  15
   Leu Leu Leu Leu Leu Leu Ala Leu Arg Gly Gln Ala Asn Thr Gly Cys
               20                  25                  30
   Tyr Gly Ile Pro Gly Met Pro Gly Leu Pro Gly Ala Pro Gly Lys Asp
           35                  40                  45
   Gly Tyr Asp Gly Leu Pro Gly Pro Lys Gly Glu Pro Gly Ile Pro Ala
       50                  55                  60
   Ile Pro Gly Ile Arg Gly Pro Lys Gly Gln Lys Gly Glu Pro Gly Leu
   65                  70                  75                  80
   Pro Gly His Pro Gly Lys Asn Gly Pro Met Gly Pro Pro Gly Met Pro
                   85                  90                  95
   Gly Val Pro Gly Pro Met Gly Ile Pro Gly Glu Pro Gly Glu Glu Gly
   100 105 110
   Arg Tyr Lys Gln Lys Phe Gln Ser Val Phe Thr Val Thr Arg Gln Thr
   115 120 125
   His Gln Pro Pro Ala Pro Asn Ser Leu Ile Arg Phe Asn Ala Val Leu
   130 135 140
   Thr Asn Pro Gln Gly Asp Tyr Asp Thr Ser Thr Gly Lys Phe Thr Cys
   145 150 155 160
   Lys Val Pro Gly Leu Tyr Tyr Phe Val Tyr His Ala Ser His Thr Ala
   165 170 175
   Asn Leu Cys Val Leu Leu Tyr Arg Ser Gly Val Lys Val Val Thr Phe
   180 185 190
   Cys Gly His Thr Ser Lys Thr Asn Gln Val Asn Ser Gly Gly Val Leu
   195 200 205
   Leu Arg Leu Gln Val Gly Glu Glu Val Trp Leu Ala Val Asn Asp Tyr
   210 215 220
   Tyr Asp Met Val Gly Ile Gln Gly Ser Asp Ser Val Phe Ser Gly Phe
   225 230 235 240
   Leu Leu Phe Pro Asp
   245
<210> 7
   <211> 253
   <212> PRT
   <213> Séquence artificielle
<220>
   <223> Sous-unité ClqC+séquence additionnelle d'acides aminés
<400> 7
   Met Asp Val Gly Pro Ser Ser Leu Pro His Leu Gly Leu Lys Leu Leu
   1 5 10 15
   Leu Leu Leu Leu Leu Leu Ala Leu Arg Gly Gln Ala Asn Thr Gly Cys
   20 25 30
   Tyr Gly Ile Pro Gly Met Pro Gly Leu Pro Gly Ala Pro Gly Lys Asp
   35 40 45
   Gly Tyr Asp Gly Leu Pro Gly Pro Lys Gly Glu Pro Gly Ile Pro Ala
       50                  55                  60
   Ile Pro Gly Ile Arg Gly Pro Lys Gly Gln Lys Gly Glu Pro Gly Leu
   65                  70                  75                  80
   Pro Gly His Pro Gly Lys Asn Gly Pro Met Gly Pro Pro Gly Met Pro
                   85                  90                  95
   Gly Val Pro Gly Pro Met Gly Ile Pro Gly Glu Pro Gly Glu Glu Gly
               100                 105                 110
   Arg Tyr Lys Gln Lys Phe Gln Ser Val Phe Thr Val Thr Arg Gln Thr
           115                 120                 125
   His Gln Pro Pro Ala Pro Asn Ser Leu Ile Arg Phe Asn Ala Val Leu
       130                 135                 140
   Thr Asn Pro Gln Gly Asp Tyr Asp Thr Ser Thr Gly Lys Phe Thr Cys
   145                 150                 155                 160
   Lys Val Pro Gly Leu Tyr Tyr Phe Val Tyr His Ala Ser His Thr Ala
                   165                 170                 175
   Asn Leu Cys Val Leu Leu Tyr Arg Ser Gly Val Lys Val Val Thr Phe
               180                 185                 190
   Cys Gly His Thr Ser Lys Thr Asn Gln Val Asn Ser Gly Gly Val Leu
           195                 200                 205
   Leu Arg Leu Gln Val Gly Glu Glu Val Trp Leu Ala Val Asn Asp Tyr
       210                 215                 220
   Tyr Asp Met Val Gly Ile Gln Gly Ser Asp Ser Val Phe Ser Gly Phe
   225                 230                 235                 240
   Leu Leu Phe Pro Asp Asp Tyr Lys Asp Asp Asp Asp Lys
                   245                 250
<210> 8
   <211> 8
   <212> PRT
   <213> Séquence artificielle
<220>
   <223> Séquence additionnelle d'acides aminés
<400> 8
   Asp Tyr Lys Asp Asp Asp Asp Lys
   1 5
<210> 9
   <211> 24
   <212> DNA
   <213> Séquence artificielle
<220>
   <223> Amorce PCR d'amplification de C1qA
<400> 9
   ctagctagca tggagggtcc ccgg 24
<210> 10
   <211> 22
   <212> DNA
   <213> Séquence artificielle
<220>
   <223> Amorce PCR d'amplification de C1qA
<400> 10
   ccggaattct caggcagatg gg 22
<210> 11
   <211> 24
   <212> DNA
   <213> Séquence artificielle
<220>
   <223> Amorce PCR d'amplification de C1qB
<400> 11
   ctagctagca tgaagatccc atgg 24
<210> 12
   <211> 21
   <212> DNA
   <213> Séquence artificielle
<220>
   <223> Amorce PCR d'amplification de C1qB
<400> 12
   cgcggatcct caggcctcca t 21
<210> 13
   <211> 24
   <212> DNA
   <213> Séquence artificielle
<220>
   <223> Amorce PCR d'amplification de C1qC
<400> 13
   ctagctagca tggacgtggg gccc 24
<210> 14
   <211> 25
   <212> DNA
   <213> Séquence artificielle
<220>
   <223> Amorce PCR d'amplification de C1qC
<400> 14
   ccggaattcc tagtcgggga agagc 25
<210> 15
   <211> 65
   <212> DNA
   <213> Séquence artificielle
<220>
   <223> Amorce PCR pour mutagénèse dirigée de C1qC
<400> 15
   ggcttcctgc tcttccccga cgattacaag gatgacgacg ataagtagga gttctgcaga 60
   tatcc 65
<210> 16
   <211> 65
   <212> DNA
   <213> Séquence artificielle
<220>
   <223> Amorce PCR pour mutagénèse dirigée de ClqC
<400> 16
   ggatatctgc agaactccta cttatcgtcg tcatccttgt aatcgtcggg gaagagcagg 60
   aagcc 65
<210> 17
   <211> 10
   <212> PRT
   <213> Séquence artificielle
<220>
   <223> Séquence additionnelle d'acides aminés
<400> 17
   Glu Gln Lys Leu Ile Ser Glu Glu Asp Leu
   1 5 10
<210> 18
   <211> 60
   <212> DNA
   <213> Séquence artificielle
<220>
   <223> Amorce PCR d'amplification de ClqC
<400> 18
   cccctcaggg gccaagccga ttacaaggat gacgacgata agaacacagg ctgctacggg 60
<210> 19
   <211> 60
   <212> DNA
   <213> Séquence artificielle
<220>
   <223> Amorce PCR d'amplification de C1qC
<400> 19
   cccgtagcag cctgtgttct tatcgtcgtc atccttgtaa tcggcttggc ccctgagggg 60
<210> 20
   <211> 6
   <212> PRT
   <213> Séquence artificielle
<220>
   <223> Etiquette 6 histidines
<400> 20
   His His His His His His
1 5
<210> 21
   <211> 51
   <212> DNA
   <213> Séquence artificielle
<220>
   <223> Amorce PCR d'amplification de C1qC
<400> 21
   gctcttcccc gaccatcacc atcaccatca ctaggagttc tgcagatatc c 51
<210> 22
   <211> 51
   <212> DNA
   <213> Séquence artificielle
<220>
   <223> Amorce PCR d'amplification de C1qC
<400> 22
   ggatatctgc agaactccta gtgatggtga tggtgatggt cggggaagag c 51
<210> 23
   <211> 253
   <212> PRT
   <213> Séquence artificielle
<220>
   <223> Sous-unité C1qC+séquence additionnelle d'acides aminés en N-terminal
<400> 23
   Met Asp Val Gly Pro Ser Ser Leu Pro His Leu Gly Leu Lys Leu Leu
   1               5                   10                  15
   Leu Leu Leu Leu Leu Leu Ala Leu Arg Gly Gln Ala Asp Tyr Lys Asp
               20                  25                  30
   Asp Asp Asp Lys Asn Thr Gly Cys Tyr Gly Ile Pro Gly Met Pro Gly
           35                  40                  45
   Leu Pro Gly Ala Pro Gly Lys Asp Gly Tyr Asp Gly Leu Pro Gly Pro
       50                  55                  60
   Lys Gly Glu Pro Gly Ile Pro Ala Ile Pro Gly Ile Arg Gly Pro Lys
   65                  70                  75                  80
   Gly Gln Lys Gly Glu Pro Gly Leu Pro Gly His Pro Gly Lys Asn Gly
                   85                  90                  95
   Pro Met Gly Pro Pro Gly Met Pro Gly Val Pro Gly Pro Met Gly Ile
               100                 105                 110
   Pro Gly Glu Pro Gly Glu Glu Gly Arg Tyr Lys Gln Lys Phe Gln Ser
           115                 120                 125
   Val Phe Thr Val Thr Arg Gln Thr His Gln Pro Pro Ala Pro Asn Ser
       130                 135                 140
   Leu Ile Arg Phe Asn Ala Val Leu Thr Asn Pro Gln Gly Asp Tyr Asp
   145                 150                 155                 160
   Thr Ser Thr Gly Lys Phe Thr Cys Lys Val Pro Gly Leu Tyr Tyr Phe
                   165                 170                 175
   Val Tyr His Ala Ser His Thr Ala Asn Leu Cys Val Leu Leu Tyr Arg
               180                 185                 190
   Ser Gly Val Lys Val Val Thr Phe Cys Gly His Thr Ser Lys Thr Asn
           195                 200                 205
   Gln Val Asn Ser Gly Gly Val Leu Leu Arg Leu Gln Val Gly Glu Glu
       210                 215                 220
   Val Trp Leu Ala Val Asn Asp Tyr Tyr Asp Met Val Gly Ile Gln Gly
   225                 230                 235                 240
   Ser Asp Ser Val Phe Ser Gly Phe Leu Leu Phe Pro Asp
                   245                 250
<210> 24
   <211> 251
   <212> PRT
   <213> Séquence artificielle
<220>
   <223> Sous-unité C1qC+6His
<400> 24
   Met Asp Val Gly Pro Ser Ser Leu Pro His Leu Gly Leu Lys Leu Leu
   1               5                   10                  15
   Leu Leu Leu Leu Leu Leu Ala Leu Arg Gly Gln Ala Asn Thr Gly Cys
               20                  25                  30
   Tyr Gly Ile Pro Gly Met Pro Gly Leu Pro Gly Ala Pro Gly Lys Asp
           35                  40                  45
   Gly Tyr Asp Gly Leu Pro Gly Pro Lys Gly Glu Pro Gly Ile Pro Ala
       50                  55                  60
   Ile Pro Gly Ile Arg Gly Pro Lys Gly Gln Lys Gly Glu Pro Gly Leu
   65                  70                  75                  80
   Pro Gly His Pro Gly Lys Asn Gly Pro Met Gly Pro Pro Gly Met Pro
                   85                  90                  95
   Gly Val Pro Gly Pro Met Gly Ile Pro Gly Glu Pro Gly Glu Glu Gly
               100                 105                 110
   Arg Tyr Lys Gln Lys Phe Gln Ser Val Phe Thr Val Thr Arg Gln Thr
           115                 120                 125
   His Gln Pro Pro Ala Pro Asn Ser Leu Ile Arg Phe Asn Ala Val Leu
       130                 135                 140
   Thr Asn Pro Gln Gly Asp Tyr Asp Thr Ser Thr Gly Lys Phe Thr Cys
   145                 150                 155                 160
   Lys Val Pro Gly Leu Tyr Tyr Phe Val Tyr His Ala Ser His Thr Ala
                   165                 170                 175
   Asn Leu Cys Val Leu Leu Tyr Arg Ser Gly Val Lys Val Val Thr Phe
               180                 185                 190
   Cys Gly His Thr Ser Lys Thr Asn Gln Val Asn Ser Gly Gly Val Leu
           195                 200                 205
   Leu Arg Leu Gln Val Gly Glu Glu Val Trp Leu Ala Val Asn Asp Tyr
       210                 215                 220
   Tyr Asp Met Val Gly Ile Gln Gly Ser Asp Ser Val Phe Ser Gly Phe
   225                 230                 235                 240
   Leu Leu Phe Pro Asp His His His His His His
                   245                 250

## Description

### Objet de l'invention

La présente invention concerne un procédé de préparation de la protéine Clq par voie recombinante.

### Arrière-plan technique

La protéine Clq appartient au complexe C1 initiateur de la voie classique d'activation du système du complément qui participe à l'immunité innée chez les mammifères. Outre Clq, le complexe initiateur comprend deux protéases à sérine, C1r et C1s, associées en un tétramère C1s-C1r-C1r-C1s. La fixation de Clq à des « signaux de danger », à savoir des complexes antigène-anticorps, notamment du type IgG, et des facteurs présents à la surface d'agents pathogènes, de cellules infectées ou de cellules apoptotiques, conduit à l'auto-activation de C1r, qui active C1s. C1s activée initie alors l'activation en cascade des autres composants du complément.

De par son rôle central dans la voie classique d'activation du complément, un déficit en Clq augmente la sensibilité de l'individu affecté aux infections microbiennes, telles que les infections à *Salmonella,* la réinfection paludéenne et les péritonites polymicrobiennes, mais provoque également une inflammation excessive ainsi qu'une autoimmunité du type lupus, ce qui indique un rôle tolérogène ou immunosuppresseur de Clq (Lu J. et al. (2008) Cellular & Molecular Immunology 5:9-21). S'agissant de cette dernière maladie, il a d'ailleurs été montré que des patients atteints de lupus érythémateux disséminé pouvaient être traités à l'aide d'un système de circulation extracorporel du sang incluant une étape de passage du sang sur une colonne d'immunoadsorption à base de protéine Clq (Pfueller B. et al. (2001) Arthritis & Rheumatism 44:1962-1963).

Ainsi, il serait intéressant, notamment dans un cadre thérapeutique ou diagnostic, ou plus largement de recherche scientifique, de disposer de sources sûres et reproductibles de protéine C1q.

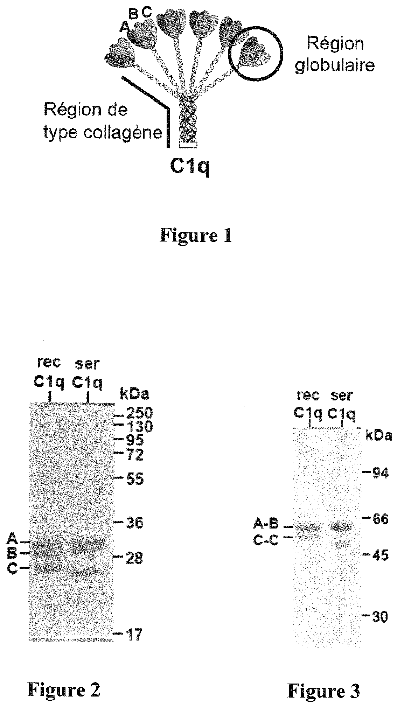

Toutefois, la structure de C1q rend sa production par voie synthétique complexe. En effet, C1q est une protéine multimérique de haut poids moléculaire (environ 460 kDa) constituée de l'association de 18 chaînes polypeptidiques : 6 sous-unités C1qA, 6 sous-unités ClqB et 6 sous-unités C1qC. Chaque sous-unité, ou chaine, contient un domaine N-terminal de type collagène et un domaine C-terminal globulaire. La sous-unité C1qA est associée à une sous-unité C1qB par la formation d'un pont disulfure et à une sous-unité ClqC de manière non-covalente pour former un hétérotrimère. Par ailleurs, les sous-unités C1qC sont reliées chacune deux à deux par la formation d'un pont disulfure. Pour chaque hétérotrimère les domaines de type collagène s'associent en une triple hélice également de type collagène et les domaines globulaires s'associent pour former une région globulaire quasi sphérique (Gaboriaud C. et al. (2003) Journal of Biological Chemistry 278:46974-46982). La forme globale de C1q est donc celle d'un « bouquet de tulipes » où les six régions globulaires émergent d'une queue formée des six triples hélices de type collagène (**Figure 1**).

De fait, à ce jour, le seul moyen d'obtenir de la protéine Clq complète est de l'extraire de sérum humain ou animal, ce qui n'est satisfaisant ni en terme de reproductibilité, ni en terme de sécurité biologique, les dérivés sanguins pouvant être contaminés par des virus, des prions ou encore des parasites unicellulaires. S'agissant de la voie recombinante, qui permettrait de résoudre ces problèmes, seuls des sous-unités ou des fragments, notamment globulaires (voir par exemple Kojouharova M. et al. (2004) Journal of Immunology 172:4351-4358), de Clq ont pu être produits.

C'est donc un objet de l'invention que de fournir un procédé reproductible d'obtention de protéine C1q complète et correctement structurée sans risque biologique associé.

### Description de l'invention

La présente invention découle de la mise en évidence inattendue, par les inventeurs, que l'adjonction d'un peptide de séquence DYKDDDDK (SEQ ID NO : 8) à l'extrémité C-terminale de la sous-unité C1qC permet, lorsqu'elle est produite par voie recombinante avec les sous-unités C1qA et C1qB dans une même cellule au cours d'une culture cellulaire *in vitro*, de générer une protéine C1q ayant une structure similaire à sa structure native.

L'objet de la présente invention est défini par les revendications 1 à 15.

Ainsi, la présente invention concerne un procédé de production d'une protéine C1q, ou d'un variant de la protéine Clq, par voie recombinante, dans lequel la protéine est récupérée à partir d'une culture in vitro de cellules exprimant une sous-unité C1qA ou un variant de la sous-unité C1qA, une sous-unité ClqB ou un variant de la sous-unité ClqB, et une sous-unité C1qC, ou un variant de la sous-unité C1qC, et dont l'une au moins des sous-unités, ou l'un au moins des variants de sous-unité, porte en outre à l'extrémité N-terminale ou C-terminale une séquence additionnelle d'acides aminés d'au moins 6 résidus dont au moins 40% sont des résidus d'acide glutamique et/ou d'acide aspartique, dans lequel un variant d'une sous-unité a une séquence d'acides aminés qui présente au moins 70% d'identité avec la séquence de la sous-unité dont elle dérive et qui permet la formation d'un variant de C1q.

La description concerne un procédé de production d'une protéine C1q, ou d'un variant de la protéine C1q, par voie recombinante, dans lequel la protéine est récupérée à partir d'une culture *in vitro* de cellules exprimant une sous-unité C1qA ou un variant de la sous-unité C1qA, une sous-unité C1qB ou un variant de la sous-unité ClqB, et une sous-unité C1qC, ou un variant de la sous-unité C1qC, et dont l'une au moins des sous-unités, ou l'un au moins des variants de sous-unité, porte en outre à l'extrémité N-terminale ou C-terminale une séquence additionnelle d'acides aminés d'au moins 6 résidus dont au moins 40% sont des résidus d'acide glutamique et/ou d'acide aspartique.

La présente invention concerne également une protéine Clq, ou variant de la protéine C1q comprenant une sous-unité C1qA ou un variant de la sous-unité C1qA, une sous-unité C1qB ou un variant de la sous-unité ClqB, et une sous-unité C1qC, ou un variant de la sous-unité C1qC, et dont l'une au moins des sous-unités, ou l'un au moins des variants de sous-unité, porte en outre à l'extrémité N-terminale ou C-terminale une séquence additionnelle d'acides aminés d'au moins 6 résidus dont au moins 40% sont des résidus d'acide glutamique et/ou d'acide aspartique, dans lequel un variant d'une sous-unité a une séquence d'acides aminés qui présente au moins 70% d'identité avec la séquence de la sous-unité dont elle dérive et qui permet la formation d'un variant de C1q.

La description concerne une protéine C1q, ou un variant de la protéine C1q, comprenant une sous-unité C1qA ou un variant de la sous-unité C1qA, une sous-unité C1qB ou un variant de la sous-unité C1qB, et une sous-unité C1qC, ou un variant de la sous-unité C1qC, et dont l'une au moins des sous-unités, ou l'un au moins des variants de sous-unité, porte en outre à l'extrémité N-terminale ou C-terminale une séquence additionnelle d'acides aminés d'au moins 6 résidus dont au moins 40% sont des résidus d'acide glutamique et/ou d'acide aspartique.

La présente invention concerne également une sous-unité ou un variant de sous-unité de la protéine C1q portant à l'extrémité N-terminale ou C-terminale une séquence additionnelle d'acides aminés d'au moins 6 résidus dont au moins 40% sont des résidus d'acide glutamique et/ou d'acide aspartique, dans laquelle un variant d'une sous-unité a une séquence d'acides aminés qui présente au moins 70% d'identité avec la séquence de la sous-unité dont elle dérive et qui permet la formation d'un variant de C1q, et dans laquelle la séquence additionnelle se situe à l'extrémité C-terminale de ladite sous-unité lorsque elle est portée par la sous-unité C1qA ou un variant de la sous-unité C1qA.

La description concerne une sous-unité, ou un variant de sous-unité de la protéine C1q, portant à l'extrémité N-terminale ou C-terminale une séquence additionnelle d'acides aminés d'au moins 6 résidus dont au moins 40% sont des résidus d'acide glutamique et/ou d'acide aspartique.

La présente invention concerne également un acide nucléique codant pour une sous-unité ou un variant de sous-unité de la protéine C1q selon l'invention.

La présente invention concerne également un vecteur comprenant un acide nucléique selon l'invention.

La présente invention concerne également une cellule comprenant un acide nucléique ou un vecteur selon l'invention.

Dans un mode de réalisation préféré de la cellule selon la description, elle comprend en outre un ou plusieurs acides nucléiques ou un ou plusieurs vecteurs codant pour les sous-unités ou variants de sous-unités complémentaires de C1q, de sorte que la cellule comprenne un ou plusieurs acides nucléiques ou vecteurs codant pour une sous-unité C1qA ou un variant de la sous-unité C1qA, une sous-unité C1qB ou un variant de la sous-unité C1qB, et une sous-unité C1qC, ou un variant de la sous-unité C1qC, et dont l'un au moins des sous-unités, ou des variants de sous-unité, porte en outre à l'extrémité N-terminale ou C-terminale une séquence additionnelle d'acides aminés d'au moins 6 résidus dont au moins 40% sont des résidus d'acide glutamique et/ou d'acide aspartique.

La présente invention concerne également l'utilisation d'un acide nucléique ou d'un vecteur selon l'invention, pour la transformation d'une cellule et la production de Clq par la cellule.

La protéine Clq appartenant au complexe C1 du système du complément est bien connue de l'homme du métier. Elle est notamment décrite dans Gaboriaud C. et al. (2003) Journal of Biological Chemistry 278:46974-46982. Comme on l'entend ici la protéine C1q désigne une protéine complète, c'est-à-dire constituée de l'association de 18 sous-unités, à savoir 6 sous-unités (ou chaine) C1qA, 6 sous-unités (ou chaine) C1qB et 6 sous-unités (ou chaine C1qC). De préférence, la protéine Clq selon l'invention est humaine. A titre d'exemple, la séquence de la sous-unité C1qA est de préférence représentée par SEQ ID NO : 1, la séquence de la sous-unité ClqB est de préférence représentée par SEQ ID NO : 3 et la séquence de la sous-unité C1qC est de préférence représentée par SEQ ID NO : 5.

Comme on l'entend ici, un « variant » d'une sous-unité dérive de la sous-unité par l'insertion, la suppression ou la substitution d'au moins un acide aminé, sous réserve que la séquence du variant présente au moins 70%, de préférence au moins 80%, plus préférablement au moins 90%, et encore plus préférablement au moins 95% d'identité avec la séquence de la sous-unité dont il dérive et que le variant de sous-unité permette de former un variant de C1q.

Comme on l'entend ici, le pourcentage d'identité entre deux séquences peptidiques peut être déterminé en réalisant un alignement optimal sur toute la longueur des séquences, en déterminant le nombre de position alignées pour lesquelles les acides aminés sont identiques dans chaque séquence et en divisant ce nombre par le nombre total d'acides aminés dans la plus longue des deux séquences. L'alignement optimal est celui qui donne le pourcentage d'identité le plus élevé entre les deux séquences. Par ailleurs, des espaces ou des trous, correspondant généralement à des acides aminés gagnés ou perdus par l'une ou l'autre des séquences au cours de l'évolution, peuvent être introduits dans l'une ou l'autre des séquences, dans une mesure que l'homme du métier sait apprécier, afin de réaliser un alignement optimal. L'homme du métier a par ailleurs à sa disposition de nombreux algorithmes ou logiciels susceptibles de réaliser un alignement optimal automatisé entre deux séquences peptidiques. On citera en particulier, à titre d'exemple, l'algorithme de Needleman & Wunsch (1970) J. Mol. Biol. 48:443-453, qui peu notamment être mis en œuvre par le logiciel EMBOSS Needle avec les paramètres par défaut suivants :
Matrix: Blosum62 ;
Gap open: 10;
Gap extend: 0.5;
Output format: pair;
End gap penalty: false;
End gap open: 10;
End gap extend: 0.5.

Comme on l'entend ici, un variant de C1q est constitué de l'association de 6 sous-unités C1qA ou variants de la sous-unité C1qA, de 6 sous-unités C1qB ou variants de la sous-unité C1qB, et de 6 sous-unités C1qC ou variants de la sous-unité C1qC et contient au moins un variant de sous-unité. De préférence, le variant de C1q selon l'invention n'est pas constitué de l'association de sous-unités dont tous les domaines globulaires ou tous les domaines de type collagène sont manquants.

De préférence, la protéine Clq, ou le variant de protéine Clq, selon l'invention ou produit selon l'invention est tel que les sous-unités de C1qA, ou les variants de sous-unités C1qA, sont reliés aux sous-unités de C1qB, ou aux variants des sous-unités de C1qB, par un pont disulfure. De préférence également, la protéine C1q, ou le variant de protéine C1q, selon l'invention ou produit selon l'invention est tel que les sous-unités de C1qC sont reliées deux à deux par un pont disulfure. De préférence également, la protéine C1q, ou le variant de protéine C1q, selon l'invention ou produit selon l'invention a une structure, notamment visible en microscopie électronique, en bouquet de tulipes. De préférence également, la protéine Clq, ou le variant de protéine Clq, selon l'invention ou produit selon l'invention présente une constante de dissociation (K_{D}) vis-à-vis du tétramère C1s-C1r-C1r-C1s qui est égale à ± 20% à celle de la protéine Clq isolée de sérum, notamment humain, mesurée dans les mêmes conditions. De préférence également, la protéine C1q, ou le variant de protéine Clq, selon l'invention ou produit selon l'invention présente une constante de dissociation (K_{D}) vis-à-vis des immunoglobulines, notamment de type G, qui est égale à ± 20% à celle de la protéine Clq isolée de sérum, notamment humain, mesurée dans les mêmes conditions. Comme on l'entend ici, la protéine Clq isolée de sérum est notamment purifiée en appliquant la méthode décrite par Arlaud GJ et al. (1979) Mol. Immunol. 16:445-450 et présentée dans la partie 4a) de l'Exemple. De préférence également, la protéine C1q, ou le variant de protéine Clq, selon l'invention ou produit selon l'invention permet, lorsqu'il est associé au tétramère C1s-C1r-C1r-C1s de provoquer l'activation de C1s. Enfin, de préférence, la protéine C1q, ou le variant de protéine C1q, produit selon l'invention peut se lier à un complexe immun ovalbumine-anticorps-anti-ovalbumine. Le complexe immun ovalbumine-anticorps-anti-ovalbumine est notamment décrit dans Arlaud GJ et al. (1979) Mol. Immunol. 16:445-450.

Comme on l'entend ici, la production par voie recombinante signifie que la protéine C1q ou le variant de C1q selon l'invention est produit par des cellules qui expriment au moins une sous-unité codée par un acide nucléique ou un vecteur qui a été introduit, notamment par transformation ou transfection, dans la cellule. De préférence, des acides nucléiques ou des vecteurs codant :
- une sous-unité C1qA ou un variant de la sous-unité C1qA, et
- une sous-unité C1qB ou un variant de la sous-unité C1qB, et
- une sous-unité C1qC, ou un variant de la sous-unité C1qC, et
dont l'une au moins des sous-unités, ou l'un au moins des variants de sous-unité, porte en outre à l'extrémité N-terminale ou C-terminale une séquence additionnelle d'acides aminés d'au moins 6 résidus dont au moins 40% sont des résidus d'acide glutamique et/ou d'acide aspartique,
ont été introduits dans les cellules selon l'invention. Dans ce cas, un, deux ou les trois séquences codant respectivement une sous-unité C1qA ou un variant de la sous-unité C1qA, une sous-unité C1qB ou un variant de la sous-unité C1qB, et une sous-unité C1qC, ou un variant de la sous-unité C1qC peuvent être présentes dans un acide nucléique ou un vecteur selon l'invention.

On préfère toutefois que chaque séquence codant respectivement une sous-unité C1qA ou un variant de la sous-unité C1qA, une sous-unité C1qB ou un variant de la sous-unité C1qB, et une sous-unité C1qC, ou un variant de la sous-unité C1qC soit présente sur un acide nucléique ou un vecteur propre. Les trois acides nucléiques ou vecteurs peuvent alors être introduits de manière concomitante ou séquentielle dans la cellule selon l'invention. Lorsque l'introduction est séquentielle, un premier acide nucléique ou vecteur est introduit, puis un deuxième acide nucléique ou vecteur est introduit, éventuellement après obtention d'une lignée cellulaire exprimant de manière stable la sous-unité ou le variant de sous-unité codé par le premier acide nucléique ou vecteur, puis un troisième acide nucléique ou vecteur est introduit, éventuellement après obtention d'une lignée cellulaire exprimant en plus de manière stable la sous-unité ou le variant de sous-unité codé par le deuxième acide nucléique ou vecteur. Lorsque l'introduction est concomitante (i) les trois acides nucléiques ou vecteurs peuvent être introduits en même temps, ou (ii) un premier acide nucléique ou vecteur est introduit puis le deuxième et le troisième acide nucléique ou vecteur sont introduits en même temps, éventuellement après obtention d'une lignée cellulaire exprimant de manière stable la sous-unité ou le variant de sous-unité codé par le premier acide nucléique ou vecteur, ou (iii) un premier et un deuxième acide nucléique ou vecteur sont introduits en même temps puis un troisième acide nucléique ou vecteur est introduit, éventuellement après obtention d'une lignée cellulaire exprimant de manière stable les sous-unités ou les variants de sous-unité codés par le premier et le deuxième acides nucléiques ou vecteurs.

Comme on l'entend ici, l'expression « culture *in vitro* » signifie que les cellules produisant la protéine Clq, ou un variant de la protéine Clq selon l'invention, sont cultivées en dehors d'un organisme vivant.

Par ailleurs, dans la culture *in vitro* selon l'invention, une même cellule produit à la fois :
- une sous-unité C1qA ou un variant de la sous-unité C1qA, et
- une sous-unité C1qB ou un variant de la sous-unité C1qB, et
- une sous-unité C1qC ou un variant de la sous-unité C1qC.

Le vecteur selon l'invention peut être de tout type. On préfère toutefois qu'il s'agisse d'un vecteur d'expression eucaryote, notamment de mammifère, comprenant donc l'ensemble des éléments nécessaires à son maintien et à l'expression de l'acide nucléique selon l'invention dans une cellule selon l'invention. On préfère ainsi notamment que le vecteur selon l'invention soit un plasmide comprenant un promoteur et un terminateur eucaryote, une origine de réplication eucaryote, et un gène de sélection eucaryote, ainsi que, de préférence, une origine de réplication procaryote et un gène de sélection procaryote, tel qu'un plasmide du type pcDNA3.1 par exemple.

Les cellules selon l'invention peuvent être de tout type. On préfère toutefois qu'il s'agisse de cellules eucaryotes, en particulier de mammifère et plus particulièrement humaine. De manière préférée, les cellules selon l'invention sont des cellules de lignée cellulaire, c'est-à-dire des cellules ayant un potentiel de division cellulaire essentiellement illimité. De manière particulièrement préférée, les cellules selon l'invention sont choisies dans le groupe constitué des cellules 293-F, HEK-293, CHO, COS-7, BHK-21, NS0 et SP2/0.

De manière préférée, les cellules selon l'invention expriment la sous-unité C1qA ou le variant de la sous-unité C1qA, la sous-unité C1qB ou le variant de la sous-unité C1qB, et la sous-unité C1qC, ou le variant de la sous-unité C1qC de manière stable. Comme on l'entend ici, une expression stable signifie que la potentialité d'expression des sous-unités, ou des variants de sous-unités, par la cellule ne disparait pas tant que la cellule conserve la potentialité générale d'exprimer des protéines et/ou que les acides nucléiques codant les sous-unités ou les variants de sous-unités sont intégrés aux chromosomes de la cellule.

De préférence, la séquence additionnelle d'acides aminés selon l'invention comprend au moins 7 ou 8 acides aminés. De préférence également, la séquence additionnelle d'acides aminés selon l'invention comprend au plus 20, 15, 10, ou 8 acides aminés. De préférence également, la séquence additionnelle d'acides aminés selon l'invention comprend au moins 50%, plus préférablement au moins 55% et encore plus préférablement au moins 60% d'acides aminés acide aspartique et/ou acide glutamique. De préférence également, la séquence additionnelle d'acides aminés selon l'invention comprend au moins 50%, plus préférablement au moins 55% et encore plus préférablement au moins 60% d'acides aminés acide aspartique. Dans le cadre de la présente description l'acide aspartique pourra également être dénommé aspartate, Asp ou D, et l'acide glutamique pourra également être dénommé glutamate, Glu, ou E. De préférence également, la séquence additionnelle d'acides aminés selon l'invention ne comprend pas de séquence codant pour une étiquette Myc. De manière particulièrement préférée la séquence d'acides aminés est la suivante : DYKDDDDK (SEQ ID NO : 8) ou EQKLISEEDL (SEQ ID NO : 17).

La séquence additionnelle d'acides aminés selon l'invention peut être placée indifféremment du côté N-terminal ou du côté C-terminal des sous-unités ou des variants de sous-unités selon l'invention, on préfère, toutefois, qu'elle soit située à l'extrémité C-terminale de l'un au moins des sous-unités ou des variants de sous-unité. De manière préférée, la séquence additionnelle d'acides aminés selon l'invention est située à l'extrémité N-terminale ou C-terminale, de préférence à l'extrémité C-terminale, de la sous-unité C1qC ou d'un variant de la sous-unité C1qC selon l'invention. On préfère également que la séquence additionnelle d'acides aminés selon l'invention soit directement accolée à l'extrémité C-terminale ou N-terminale des sous-unités ou des variants de sous-unités selon l'invention, c'est-à-dire qu'il n'y ait pas de séquence intermédiaire ou entre la séquence additionnelle et la séquence des sous-unités ou des variants de sous-unité selon l'invention. De manière tout particulièrement préférée, la séquence additionnelle d'acides aminés selon l'invention est directement accolée à l'extrémité C-terminale de la sous-unité C1qC ou d'un variant de la sous-unité C1qC selon l'invention et on préfère, dans ce cadre, que la séquence de la sous-unité C1qC portant la séquence additionnelle d'acides aminés selon l'invention soit représentée par SEQ ID NO : 7. Par ailleurs, de manière préférée la séquence additionnelle d'acides aminés selon l'invention est clivable, c'est-à-dire qu'elle peut être éliminée, en totalité ou en partie, de la protéine Clq après sa production selon l'invention. En particulier, lorsqu'elle est clivable, la séquence additionnelle d'acides aminés selon l'invention contient un site de coupure pour une protéase. Comme l'homme du métier le comprendra bien, une partie des acides aminés constitutifs du site de reconnaissance de la protéase peuvent se trouver sur la portion de la sous-unité ou du variant de sous-unité jouxtant la séquence additionnelle d'acides aminés selon l'invention. Par ailleurs, à titre d'exemple de protéase, on peut citer l'entérokinase qui provoque une coupure après la lysine (K) du site de coupure DDDK.

La protéine C1q, ou le variant de la protéine C1q, peut être aisément récupérée à partir de la culture selon l'invention, puis éventuellement être purifiée, à l'aide de nombreuses techniques bien connues de l'homme du métier. De préférence, la protéine C1q, ou le variant de la protéine C1q, est récupérée à partir du surnageant de la culture selon l'invention, c'est-à-dire la partie non cellulaire de la culture, plus préférablement comme cela est indiqué dans les Exemples qui suivent.

De préférence, la culture *in vitro* selon l'invention contient de la vitamine C, notamment à une concentration de 20 à 200 µg/ml, plus préférablement à une concentration de 80 à120 µg/ml et encore plus préférablement à une concentration de l'ordre de 100 µg/ml.

L'invention sera davantage explicitée, de manière non limitative, à l'aide des figures et de l'Exemple qui suit.

### Description des figures

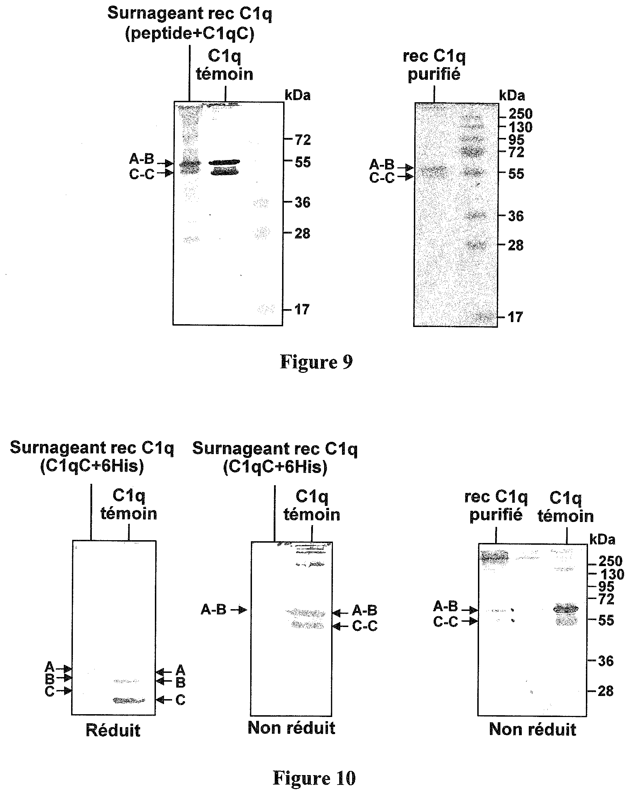

Figure 1
   La figure 1 représente la structure quaternaire, dite en « bouquet de tulipes » de la protéine C1q, résultant de l'association de 6 sous-unités C1qA, 6 sous-unités C1qB ainsi que 6 sous-unités C1qC, et distinguant les régions globulaires et de type collagène.
Figures 2 et 3
   Les figures 2 et 3 représentent l'analyse par électrophorèse sur gel de polyacrylamide-SDS de C1q recombinant selon l'invention (rec) et dérivé du sérum (ser) en conditions réductrices (figure 2) et non réductrices (figure 3).
Figures 4 et 5

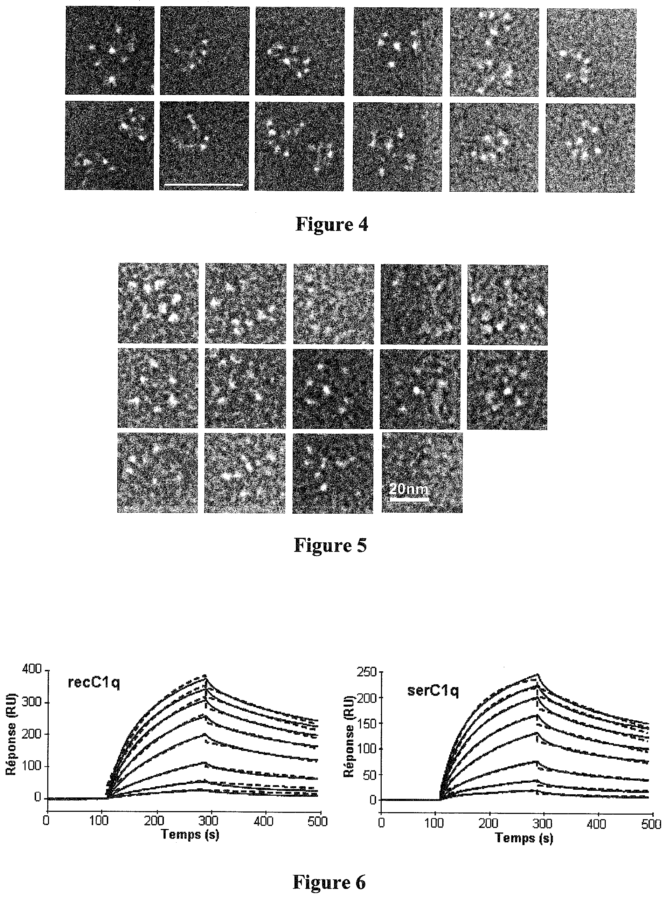

   Les figures 4 et 5 représentent l'analyse par microscopie électronique de C1q recombinant selon l'invention après coloration négative au silicotungstate de sodium 2% (figure 4) ou au molybdate d'ammonium 2% (figure 5). Les barres blanches représentent 20 nm.
Figures 6 et 7

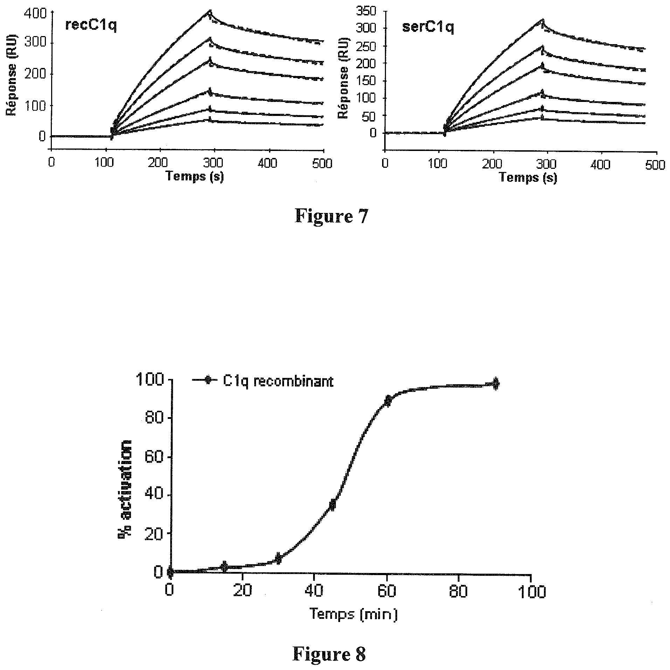

   Les figures 6 et 7 représentent la réponse mesurée par résonance plasmonique de surface (axe des ordonnées, en unité de résonance (RU)) de la mise en contact de Clq recombinant et Clq sérique immobilisés avec des concentrations variables du tétramère C1s-C1r-C1r-C1s (1, 2, 4, 8, 12, 16, 20 et 24 nM) (figure 6) ou d'IgG humaines (12.5, 25, 50, 100, 150 et 200 nM) (figure 7) en fonction du temps (axe des abscisses, en secondes). Les courbes expérimentales sont en traits pleins et les courbes ajustées en pointillés.
Figure 8
   La figure 8 représente le pourcentage d'activation de la sous-unité C1s (axe des ordonnées) en fonction de temps (axe des abscisses, en minutes) dans le complexe C1 reconstitué à partir de Clq recombinant selon l'invention et du tétramère C1s-C1r-C1r-C1s proenzyme.
Figure 9
   Le panneau de gauche de la figure 9 représente l'analyse par électrophorèse sur gel en conditions non réductrices et marquage de Western avec un anticorps anti-C1q plasmatique du surnageant de culture (1ml) de Clq recombinant contenant la chaîne peptide+C1qC. Témoin : 1.5 µg de C1q plasmatique

Le panneau de droite de la figure 9 représente l'analyse par électrophorèse sur gel en conditions non réductrices de Clq recombinant purifié (4,8µg) contenant la chaîne peptide+C1qC

### Figure 10

La figure 10 représente l'analyse par électrophorèse sur gel et marquage de Western avec un anticorps anti-Clq plasmatique du surnageant de culture (2ml) de C1q recombinant contenant la chaîne C1qC+6His en conditions réductrices (panneau de gauche) et non réductrices (panneau du centre). Témoin : 2 µg de C1q plasmatique

Le panneau de droite de la figure 10 représente l'analyse par électrophorèse sur gel en conditions non réductrices et marquage de Western avec un anticorps anti-C1q plasmatique de la totalité de C1q recombinant purifié contenant la chaîne C1qC+6His.

### EXEMPLES

### Exemple 1

### 1. Clonage de Clq recombinant

### a) Préparation des inserts

Des clones abritant les séquences codant les sous-unités C1qA, C1qB et C1qC, respectivement définies par les identifiants de la base de données *GenBank* NM 015991, NM 000491 et NM 172369 ont été obtenues chez *Origene* (Rockville, MD, USA) (voir le **Tableau 1).**

**Tableau 1**

| **Clones ORIGENE** |
|---|
| C1qA-pcMV6-Ac (NM 015991) |
| C1qB-pc MV6-XL4 (NM 000491) |
| C1qC-pc MV6-XL4 (NM 172369) |

Les clones C1qA, C1q B et C1qC ont été amplifiés par transformation avec 5ng d'ADN en bactéries *Escherichia coli* DH5a (Invitrogen) puis purifiés à l'aide du kit *QIAPrep Miniprep* de Qiagen.

Les séquences codantes des sous-unités C1qA, C1qB et C1qC ont ensuite été amplifiées à l'aide des oligonucléotides amorces présentés dans le **Tableau 2** à l'aide du protocole suivant :

**Mélange réactionnel :**

| | |
|---|---|
| - matrice (clone) : | 200 ng |
| - MgSO4 : | 2 mM |
| - dNTPs : | 400 µM |
| - Amorces : | 100 pmoles |
| - Vent polymerase (New England Biolabs) : | 2 U |

### Cycles de PCR :

1. 95°C 2 min
2. 95°C 1 min
3. 60°C 1 min
4. 72°C 1 min
   Répéter 35 fois les cycles 2 à 4
5. 72°C 5 min

**Tableau 2**

| **Chaîne** | **Amorce amont (5'-3')** | **Amorce aval (5'-3')** |
|---|---|---|
| **C1qA** | CTAGCTAGCATGGAGGGTCCCCGG (SEQ ID NO : 9) | CCGGAATTCTCAGGCAGATGGG (SEQ ID NO : 10) |
| **C1qB** | CTAGCTAGCATGAAGATCCCATGG (SEQ ID NO : 11) | CGCGGATCCTCAGGCCTCCAT (SEQ ID NO : 12) |
| **C1qC** | CTAGCTAGCATGGACGTGGGGCCC (SEQ ID NO : 13) | CCGGAATTCCTAGTCGGGGAAGAGC (SEQ ID NO : 14) |

Les produits d'amplification PCR sont ensuite purifiés à l'aide du kit *QIAquick PCR purification kit* (Qiagen) puis coupés à l'aide des enzymes de restriction *Nhe*I et *Eco*RI pour C1qA et C1qC, et *Nhe*I et *Bam*HI pour C1qB. Les fragments coupés sont enfin purifiés à nouveau à l'aide du *QIAquick PCR purification kit.*

### b) Préparation des vecteurs

Des vecteurs pcDNA3.1. (Invitrogen) incorporant respectivement 3 gènes de résistance aux antibiotiques distincts (voir le **Tableau 3**) ont été utilisé pour accueillir les inserts.

Les vecteurs sont coupés par les enzymes suivantes (voir également le **Tableau 3)** :
- *Nhe*I et *Eco*RI pour pcDNA3.1 et pcDNA3.1/Zéomycine
- *Nhe*I et *Bam*HI pour pcDNA3.1/Hygromycine

Les vecteurs coupés ont été soumis à une déphosphorylation classique à l'aide du kit *Shrimp alkaline phosphatase* (Roche) puis ont été purifiés à l'aide du *QIAquick DNA purification kit* (Qiagen).

**Tableau 3 (Néomycine = G418 = Généticine)**

| **Sous-unité** | **Sites de coupure** | **Vecteurs d'expression (gène de résistance)** |
|---|---|---|
| C1qA | *Nhe*I - *Eco*RI | pcDNA3.1 (Néomycine) |
| C1qB | *Nhe*I - *Bam*HI | pcDNA3.1 (Hygromycine) |
| C1qC | *Nhe*I - *Eco*RI | pcDNA3.1 (Zéocine) |

### c) Clonage

Le *Rapid DNA ligation kit* (Roche) a été utilisé pour ligaturer les inserts dans les vecteurs coupés. Le vecteur et l'insert ont été mélangés dans un rapport molaire vecteur / insert = 1/5 soit 50 ng de vecteur (environ 5300 pb) pour 35 ng d'insert (environ 700 pb). Le mélange de ligature est incubé 30 minutes à température ambiante et des bactéries *E. coli* DH5a sont transformées avec 2,5 µl du mélange. L'ADN des bactéries transformées et résistantes à l'antibiotique adéquat est extrait puis criblé à l'aide des enzymes de restrictions utilisées pour le clonage. L'ADN plasmidique des bactéries sélectionnées est alors séquencé.

### d) Insertion de la séquence DYKDDDDK (SEQ ID NO : 8) en C-terminal

La séquence DYKDDDDK (SEQ ID NO : 8) a été insérée à l'extrémité C-terminale de la sous-unité C1qC par mutagénèse dirigée à l'aide du kit Quickchange (Agilent) pour former la sous-unité C1qC+peptide.

Brièvement, une amplification du plasmide pcDNA3.1.C1qC est réalisée par PCR à l'aide de deux amorces complémentaires incorporant la séquence codant le peptide à insérer et se liant respectivement aux deux brins du plasmide. Pour ceci, un mélange réactionnel comprenant 200 ng du plasmide pcDNA3.1.C1qC ainsi que 225 ng d'un oligonucléotide de séquence GGCTTCCTGCTCTTCCCCGACGATTACAAGGATGACGACGATAAGTAGGAGTTCT GCAGATATCC (SEQ ID NO: 15) et d'un oligonucléotide de séquence GGATATCTGCAGAACTCCTACTTATCGTCGTCATCCTTGTAATCGTCGGGGAAGA GCAGGAAGCC (SEQ ID NO : 16) est réalisé (les parties soulignées correspondent à la séquence du peptide à insérer). 5 µl de « *Quick solution* », 1 µl de « *dNTP mix* » et 1 µl de Pfu *Turbo DNA polymérase* (2,5 U/µl) (Agilent) sont ajoutés au mélange avant amplification.

L'amplification est alors réalisée dans les conditions suivantes :

| | | |
|---|---|---|
| Cycle 1 | 95°C | 1 min |
| Cycle 2 | 95°C | 50 sec |
| Cycle 3 | 60°C | 50 sec |
| Cycle 4 | 68°C | 12 min |
| Répéter 18 fois les cycles 2 à 4 | | |
| Cycle 5 | 68°C | 7 min |

2 traitements consécutifs de 1 heure avec 1 µl de nucléase *Dpn*I (10 U/µl) sont alors réalisés afin de détruire les plasmides matrices pcDNA3.1.C1qC, puis 2 µl du mélange sont utilisés pour transformer des bactéries *E. coli* XL10 Gold (Agilent).

L'ADN des bactéries transformées et résistantes à la zéocine est extrait puis criblé à l'aide de l'enzyme de restriction EcoRI. L'ADN plasmidique des bactéries sélectionnées est alors séquencé.

Les séquences des 3 chaînes de Clq obtenues sont représentées ci-après :
- C1qA
- ClqB
- ClqC+peptide

La séquence du peptide est soulignée et les séquences des chaînes matures (après coupure du peptide signal) sont indiquées en gras.

### 2. Transfections Stables de cellules 293 F

### a) Génération de lignées stables produisant 1 chaîne (C1qA, C1qB ou C1qC+peptide)

Des cellules 293-F (Invitrogen) en suspension cultivées en milieu sans sérum FreeStyle 293 Expression Medium (Invitrogen) ont été utilisées.

### Transfection :

Le mélange suivant a été réalisé au jour J :
- 30.10⁶ cellules 293-F en suspension dans 30 ml de milieu sans sérum FreeStyle 293 Expression Medium (Invitrogen)
- 30 µg ADN en milieu Optimem (Invitrogen) (pcDNA3.1 contenant l'ADN codant pour la chaîne d'intérêt)
- 60 µl de 293-Fectin (Invitrogen) en milieu Optimem (Invitrogen)
   Cellules en milieu 293F et (ADN + transfectant).

### Etablissement des lignées stables

J+3 : changement de milieu (prélèvement pour vérification de la production des protéines par électrophorèse et Western blot) avec ajout :
- de l'antibiotique de sélection approprié :
   G418 (néomycine) 400 µg/ml final (C1qA)
   Hygromycine 100 µg/ml final (ClqB)
   Zéocine 10 µg/ml final (C1qC ou C1qC+peptide)
- de Vitamine C 100 µg/ml final (pour le bon repliement des hélices de type collagène).

La culture a été maintenue avec passage 2 fois par semaine (volume ajusté en fonction du nombre de cellules vivantes de manière à rester entre 1 et 1,2.10⁶ cellules vivantes / ml) jusqu'à ce que les cellules résistantes recommencent à se multiplier (généralement à J+21).

A J+38 : vérification de la production de protéine par électrophorèse sur gel et marquage de Western avec des anticorps anti-chaîne C1qA, C1qB, C1qC ou anti-peptide.

A J+40 : les cellules sont congelées.

### b) Génération de lignées stables produisant 2 chaînes de C1q

Ce protocole a été utilisé pour générer d'emblée une lignée produisant les chaînes C1qA+C1qB, mais il peut être appliqué pour C1qA+C1qC et C1qB+C1qC. La transfection a été réalisée comme indiqué ci-dessus en mettant en présence 30.10⁶ cellules avec 30 µg d'ADN total (15 µg d'ADN plasmidique codant pour chacune des 2 chaînes). La sélection de transfectants stables est réalisée avec le couple d'antibiotiques approprié (chacun à la même concentration que s'il était utilisé seul).

Alternativement, il est possible de transfecter une lignée stable produisant déjà une chaîne par l'ADN codant pour une seconde chaîne dans les mêmes conditions (transfection séquentielle). Ce dernier protocole a été utilisé pour générer des lignées stables produisant C1qA et ClqC+peptide ainsi que ClqB et ClqC+peptide.

### c) Génération de lignées stables produisant les 3 chaînes de C1q :

Le même protocole qu'indiqué ci-dessus a été utilisé pour transfecter une lignée stable produisant déjà 2 chaînes avec l'ADN codant pour la troisième chaîne puis pour sélectionner les transfectants stables en présence des 3 antibiotiques.

Alternativement, il est possible de transfecter une lignée stable produisant 1 chaîne par l'ADN codant pour les 2 autres chaînes (30 µg d'ADN total contenant 15 µg codant pour chacune des 2 chaînes), la sélection s'effectuant en présence des 3 antibiotiques.

La vérification de la présence des 3 chaînes est effectuée en marquage de Western avec des anticorps anti-chaîne C1qA, ClqB, ClqC et anti-peptide.

Les inventeurs ont ainsi produit les lignées stables suivantes :
- C1qA (A)
- C1qB (B)
- C1qC+peptide (C)
- C1qAB (AB) (transfection concomitante par pcDNA3.1.C1qA et pcDNA3.1.C1qB)
- C1qC+peptide + C1qA **(C+A)**
- C1qC+peptide + C1qB **(C+B)**
- C1qAB + C1qC+peptide **(AB+C)** (transfection concomitante par pcDNA3.1.C1qA et pcDNA3.1.C1qB dans une première étape puis par C1qC+peptide dans une deuxième étape)
- C1qC+peptide + C1q A + C1q B **(C+A+B)**
- C1qC+peptide + C1qB + C1qA **(C+B+A)**

### 3. Production de la protéine recombinante

La lignée stable C1qAB + C1qC+peptide a été mise en culture dans le milieu sans sérum FreeStyle 293 Expression Medium (Invitrogen).

A partir de J+10, des prélèvements du surnageant ont été effectués toutes les 72H pendant 2 à 3 semaines.

### 4. Purification

Une purification en deux étapes a été mise en œuvre. Dans une première étape une purification du surnageant de culture à l'aide de complexes immuns ovalbumine-anti-ovalbumine a été réalisée de manière semblable à ce qui a été précédemment décrit pour la purification de C1q à partir de sérum humain par Arlaud GJ et al. (1979) Mol. Immunol. 16:445-450. Puis, dans une deuxième étape une purification par chromatographie d'affinité a été réalisée.

### a) Adsorption du surnageant de culture sur des complexes immuns

### Etape 1 : Conditionnement des complexes immuns (solution stock à 10 mg/ml)

- Centrifugation 8 min à 9000 rpm (4°C) et reprise du culot par du tampon Tris 20mM, NaCl 120 mM, CaCl₂ 2,5 mM pH 7,4 ;
- Incubation 30 min à 30°C avec du DFP 2mM (inhibiteur de protéases) ; puis
- Lavage avec du tampon Tris 20 mM, NaCl 120 mM, CaCl₂ 2,5 mM pH 7,4.

### Etape 2 : Fixation de C1q et lavages

- pH du surnageant de culture ajusté à 7 ;
- Complexes immuns (IC) lavés mis en suspension dans le surnageant (exemple : 800ml de surnageant pour 57 mg d'IC) en présence de CaCl₂ 2 mM et incubation 45 min à 0°C ;
- Elimination du surnageant par centrifugation (10 min, 10000 rpm) ;
- Lavage des complexes immuns avec 30ml de tampon Tris 20 mM, NaCl 120 mM, CaCl₂ 2,5 mM pH 7,4 (3 fois).

### Etape 3 : Extractions de C1q

- Extraction de Clq par du tampon Tris 50 mM, NaCl 0,7 M pH 10 (2 extractions avec 5 ml puis 1 extraction avec 2,5 ml) ;
- Analyse par électrophorèse sur gel de polyacrylamide (10%)-SDS des échantillons des différentes étapes (perfusat, lavages, extractions) en conditions non réductrices ;
- Rassemblement des 3 extractions et dialyse contre du tampon Tris 50 mM, NaCl 150 mM pH 7,4.

### b) Chromatographie sur résine anti-peptide

- 2,5 ml de résine anti-peptide M2 affinity Gel (SIGMA ref: A2220) sont lavées en tampon Tris 50mM, NaCl 150 mM pH 7,4 à 7,5 ml/h (5 volumes de colonne) ;
- 3 lavages séquentiels par 1 volume de colonne de glycine 0,1 M pH 3,5 ;
- Lavage par 5 volumes de tampon Tris 50 mM, NaCl 150 mM pH 7,4 ;
- Dépôt de C1q extrait des complexes immuns en « boucle » sur la nuit à 7,5 ml/h, soit 7 passages environ ;
- Lavages avec 5 volumes de colonne ;
- Elution avec 2 x 1 volume de colonne avec du peptide à 100 µg/ml en tampon Tris 50 mM, NaCl 150 mM pH 7,4 ;
- Analyses des différentes fractions par électrophorèse sur gel 14% en conditions non réductrices ;
- Rassemblement des fractions contenant C1q ;
- Dialyse contre du tampon triéthanolamine-HCl 50 mM, NaCl 145 mM pH 7,4
- Concentration (cellule Amicon Ultra à seuil de coupure à 30 kDa) jusqu'à atteindre une concentration de 0,4-0,5 mg/ml.

### 5. Caractérisation de la protéine recombinante purifiée

### a) Analyse par électrophorèse sur gel de polyacrylamide en présence de SDS

En conditions réductrices (**Figure 2**) : obtention des 3 chaînes A, B et C+peptide. On vérifie bien que cette dernière migrant plus lentement que la chaîne C de C1q dérivé du sérum du fait d'une masse un peu plus importante.

En conditions non réductrices (**Figure 3**) : obtention de 2 bandes, correspondant à C-C et A-B.

### b) Séquençage N-terminal

Analyse après transfert sur membrane de PVDF :
- chaîne A : Glu-Asp-Leu-(Cys)-Arg-Ala-Pro
- chaîne B : Glutamine N-terminale bloquée par formation d'un cycle pyroglutamate, comme décrit pour C1q dérivé du sérum humain par Reid & Thompson (1978) Biochem. J. 173: 863-868)
- chaîne C : Asn-Thr-Gly-(Cys)-Tyr-Gly-Ile-Pro

### b) Caractérisation par spectrométrie de masse

Chaîne A : 27278 ± 10 Da
Chaîne B : 25497 ± 55 Da
Chaîne C : 24898 ± 164 Da

Les masses des trois chaînes recombinantes apparaissent du même ordre de grandeur que celles de leurs homologues issues de C1q sérique, compte tenu de l'addition du peptide sur la chaîne C, et semblent donc comporter les modifications post-traductionnelles de glycosylation et d'hydroxylation.

### c) Caractérisation par microscopie électronique

Les **Figures 4 et 5** représentent respectivement l'analyse par microscopie électronique de C1q recombinant selon l'invention après coloration négative au silicotungstate de sodium 2% et au molybdate d'ammonium 2%. La structure caractéristique en bouquet de tulipes est clairement visible dans les deux cas.

### d) Propriétés d'interaction (analyse par résonance plasmonique de surface)

C1q recombinant et C1q sérique ont été immobilisés sur une sensorchip CM5 (GE Healthcare) et des concentrations variables du tétramère C1s-C1r-C1r-C1s (1, 2, 4, 8, 12, 16, 20 et 24 nM) (**Figure 6**) ou d'IgG humaines (12,5, 25, 50, 100, 150 et 200 nM) (**Figure 7**) ont été injectées à leur surface.

On observe des réactivités semblables pour la forme recombinante et la forme sérique.

Ceci est confirmé par les constantes cinétiques d'association et de dissociation, calculées à l'aide du logiciel Biaeval (GE Healthcare) et présentées dans le **Tableau 4** suivant :

| C1r2-C1s2 | **recC1q** | **serC1q** | IgG | **recC1q** | **serC1q** |
|---|---|---|---|---|---|
| *k*ₐ (M⁻¹ s⁻¹) | 6.07 10⁵ | 6.81 10⁵ | *k*ₐ (M⁻¹ s⁻¹) | 1.92 10⁴ | 1.95 10⁴ |
| *k*_{d} (s⁻¹) | 1.96 10⁻³ | 2.12 10⁻³ | *k*_{d} (s⁻¹) | 1.05 10⁻³ | 1.2 10⁻³ |
| ***K*_{D} (nM)** | **3,22** | **3,12** | ***K*_{D} (nM)** | **54,7** | **61,3** |

### e) Propriétés d'autoactivation

Le complexe C1 est reconstitué à partir de Clq et du tétramère C1s-C1r-C1r-C1s proenzyme. L'incubation à 37°C du complexe reconstitué entraîne l'autoactivation de C1r qui à son tour active C1s. L'activation de C1s est mesurée par électrophorèse sur gel de polyacrylamide-SDS en conditions réductrices, suivie d'un transfert sur membrane de nitrocellulose et immunorévélation avec un anticorps anti-C1s. Les résultats sont présentés dans la **Figure 8****.**

On observe que Clq recombinant présente une courbe d'activation similaire à celle obtenue habituellement pour Clq sérique.

### Exemple 2

### 1. Clonage de deux autres chaînes de C1qC modifiées par adjonction d'une séquence additionnelle d'acides aminés

### Insertion de la séquence DYKDDDDK (SEQ ID NO : 8) en N-terminal

La séquence DYKDDDDK (SEQ ID NO : 8) a été insérée à l'extrémité N-terminale de la sous-unité ClqC par mutagénèse dirigée à l'aide du kit Quickchange (Agilent) pour former la sous-unité peptide+C1qC.

Brièvement, une amplification du plasmide pcDNA3.1.C1qC est réalisée par PCR à l'aide de deux amorces complémentaires incorporant la séquence additionnelle d'acides aminés à insérer et se liant respectivement aux deux brins du plasmide. Pour ceci, un mélange réactionnel comprenant 200 ng du plasmide pcDNA3.1.C1qC ainsi que 225 ng d'un oligonucléotide de séquence CCCCTCAGGGGCCAAGCCGATTACAAGGATGACGACGATAAGAACACAGGCTGC TACGGG (SEQ ID NO : 18) et d'un oligonucléotide de séquence CCCGTAGCAGCCTGTGTTCTTATCGTCGTCATCCTTGTAATCGGCTTGGCCCCTGA GGGG (SEQ ID NO : 19) est réalisé (les parties soulignées correspondent à la séquence du peptide à insérer). 5 µl de « *Quick solution* », 1 µl de « *dNTP mix* » et 1 µl de Pfu *Turbo DNA polymérase* (2,5 U/µl) (Agilent) sont ajoutés au mélange avant amplification.

L'amplification est alors réalisée dans les conditions suivantes :

| | | |
|---|---|---|
| Cycle 1 | 95°C | 1 min |
| Cycle 2 | 95°C | 50 sec |
| Cycle 3 | 60°C | 50 sec |
| Cycle 4 | 68°C | 12 min |
| Répéter 18 fois les cycles 2 à 4 | | |
| Cycle 5 | 68°C | 7 min |

2 traitements consécutifs de 1 heure avec 1 µl de nucléase *Dpn*I (10 U/µl) sont alors réalisés afin de détruire les plasmides matrices pcDNA3.1.C1qC, puis 2 µl du mélange sont utilisés pour transformer des bactéries *E. coli* XL10 Gold (Agilent).

L'ADN plasmidique des bactéries transformées et résistantes à la zéocine est extrait puis criblé par séquençage.

### Insertion de la séquence 6His en C-terminal

La séquence HHHHHH (SEQ ID NO : 20) a été insérée à l'extrémité C-terminale de la sous-unité ClqC par mutagénèse dirigée à l'aide du kit Quickchange (Agilent) pour former la sous-unité ClqC+6His.

Brièvement, une amplification du plasmide pcDNA3.1.C1qC est réalisée par PCR à l'aide de deux amorces complémentaires incorporant la séquence codant le peptide 6His à insérer et se liant respectivement aux deux brins du plasmide. Pour ceci, un mélange réactionnel comprenant 200 ng du plasmide pcDNA3.1.C1qC ainsi que 225 ng d'un oligonucléotide de séquence
GCTCTTCCCCGACCATCACCATCACCATCACTAGGAGTTCTGCAGATATCC (SEQ ID NO: 21) et d'un oligonucléotide de séquence GGATATCTGCAGAACTCCTAGTGATGGTGATGGTGATGGTCGGGGAAGAGC (SEQ ID NO : 22) est réalisé (les parties soulignées correspondent à la séquence des 6 histidines à insérer). 5 µl de « *Quick solution* », 1 µl de « *dNTP mix* » et 1 µl de Pfu *Turbo DNA polymérase* (2,5 U/µl) (Agilent) sont ajoutés au mélange avant amplification.

L'amplification par PCR, le traitement par *DpnI*, la transformation des bactéries, l'extraction et le criblage de l'ADN plasmidique des bactéries transformées résistantes à la zéocine sont réalisés dans les conditions décrites ci-dessus pour peptide+C1qC.

Les séquences des deux nouvelles chaînes C de C1q obtenues sont représentées ci-après :
- peptide+C1qC
- C1qC+6xHis

La séquence de la séquence additionnelle d'acides aminés ou de l'étiquette 6His est soulignée et les séquences des chaînes matures (après coupure du peptide signal) sont indiquées en gras.

### 2. Transfections Stables de cellules 293 F

### Génération de lignées stables produisant les 3 chaînes de C1q:

Le même protocole qu'indiqué dans l'Exemple 1 a été utilisé pour transfecter une lignée stable produisant déjà les chaînes A et B avec l'ADN codant pour chacun des deux nouveaux variants de la chaîne C (peptide+C1qC et C1qC+6His), puis pour sélectionner les transfectants stables en présence des 3 antibiotiques.

La vérification de la présence des 3 chaînes est effectuée en marquage de Western avec un anticorps anti- C1q plasmatique (**Figures 9 et 10**). On peut noter que dans le cas de C1q recombinant contenant la chaîne C+6His, les trois chaînes sont détectées dans le surnageant de culture en conditions réductrices, mais il n'y a pas d'évidence de présence du dimère C-C en conditions non réductrices, ce qui suggère un mauvais repliement de la protéine. Le dimère C-C est visible dans le cas de C1q recombinant contenant la chaîne peptide+C1qC.

Les inventeurs ont ainsi produit les lignées stables supplémentaires suivantes :
- C1qAB + peptide+C1qC (AB+C) (transfection concomitante par pcDNA3.1.C1qA et pcDNA3.1.C1qB dans une première étape puis par peptide+C1qC dans une deuxième étape)
- C1qAB + C1qC+6His **(AB+C)** (transfection concomitante par pcDNA3.1.C1qA et pcDNA3.1.C1qB dans une première étape puis par ClqC+6His dans une deuxième étape)

### 3. Production de la protéine recombinante

Les deux nouvelles lignées stables obtenues ont été mises en culture comme décrit dans l'Exemple 1.

### 4. Purification

La protéine C1q recombinante contenant une chaîne C avec la séquence additionnelle d'acides aminés en N-terminal (peptide+C1qC) a été purifiée comme la protéine recombinante contenant une chaîne C avec la séquence additionnelle d'acides aminés en C-terminal (C1qC+peptide), en deux étapes (adsorption du surnageant de culture sur des complexes immuns et chromatographie sur résine anti-peptide)

Après purification à partir de 470 ml de surnageant de culture, 36 µg de C1q recombinant purifié (**Figure 9**) ont été obtenus, ce qui représente un rendement d'environ 80 µg/litre, soit 10% de la quantité obtenue dans les mêmes conditions avec C1q recombinant contenant la chaîne C avec la séquence additionnelle d'acides aminés en C-terminal (800 µg/litre).

La présence de la séquence additionnelle d'acides aminés en N-Terminal de la chaîne C de C1q permet la production de C1q recombinant mais entraîne une diminution du rendement de production de C1q recombinant par rapport à la configuration où le peptide est placé en C-terminal.

La protéine Clq recombinante contenant une chaîne C avec une étiquette 6His en C-Terminal (C1qC+6His) a été purifiée en deux étapes :
*Etape 1* : adsorption du surnageant de culture sur des complexes immuns (identique à celle utilisée pour la protéine avec ClqC+peptide). Les 3 extractions sont rassemblées et dialysées contre du tampon phosphate salin (PBS) contenant 10 mM imidazole. La quantité de protéines extraites est très faible.
*Etape 2 :* chromatographie d'affinité sur ions nickel immobilisés :
   - 1,5 ml de résine HIS-Select HF Nickel Affinity Gel (SIGMA ref: H0537) sont lavés en PBS + 10 mM imidazole
   - dépôt des fractions extraites des complexes immuns
   - lavage par 20 ml PBS + 10 mM imidazole
   - élution en PBS + 300 mM imidazole
   - la totalité de l'éluat est utilisée pour une analyse en marquage de Western avec un anticorps anti-C1q plasmatique (**Figure 10**)

La quantité obtenue dans ces conditions à partir de 500 ml de surnageant est inférieure au µg, ce qui confirme l'analyse des surnageants de culture en conditions non réductrices, révélant une quantité infime de matériel correctement replié.

La très grande majorité de Clq recombinant produit avec une étiquette 6His en C-Terminal de la chaîne C1qC n'est pas correctement repliée.

**Tableau des séquences :**

| **SEQ ID NO :** | **Description** |
|---|---|
| 1 | Séquence peptidique de C1qA sans peptide signal |
| 2 | Séquence peptidique de C1qA avec peptide signal |
| 3 | Séquence peptidique de C1qB sans peptide signal |
| 4 | Séquence peptidique de C1qB avec peptide signal |
| 5 | Séquence peptidique de C1qC sans peptide signal |
| 6 | Séquence peptidique de C1qC avec peptide signal |
| 7 | Séquence peptidique de C1qC avec peptide signal et avec la séquence additionnelle d'acides aminés |
| 8 | Séquence additionnelle d'acides aminés |
| 9 | Amorce PCR d'amplification de C1qA |
| 10 | Amorce PCR d'amplification de C1qA |
| 11 | Amorce PCR d'amplification de C1qB |
| 12 | Amorce PCR d'amplification de C1qB |
| 13 | Amorce PCR d'amplification de C1qC |
| 14 | Amorce PCR d'amplification de C1qC |
| 15 | Amorce PCR pour mutagenèse dirigée de C1qC |
| 16 | Amorce PCR pour mutagenèse dirigée de C1qC |
| 17 | Séquence additionnelle d'acides aminés |
| 18 | Amorce PCR d'amplification de C1qC |
| 19 | Amorce PCR d'amplification de C1qC |
| 20 | Etiquette six histidines (6His) |
| 21 | Amorce PCR d'amplification de C1qC |
| 22 | Amorce PCR d'amplification de C1qC |
| 23 | Séquence peptidique de C1qC avec peptide signal et avec la séquence additionnelle d'acides aminés du côté N-terminal |
| 24 | Séquence peptidique de C1qC avec peptide signal et avec la séquence 6His du côté C-terminal |

## Revendications

1. Procédé de production d'une protéine Clq, ou d'un variant de la protéine C1q, par voie recombinante, dans lequel la protéine est récupérée à partir d'une culture *in vitro* de cellules exprimant une sous-unité C1qA ou un variant de la sous-unité C1qA, une sous-unité ClqB ou un variant de la sous-unité C1qB, et une sous-unité C1qC, ou un variant de la sous-unité C1qC, et dont l'une au moins des sous-unités, ou l'un au moins des variants de sous-unité, porte en outre à l'extrémité N-terminale ou C-terminale une séquence additionnelle d'acides aminés d'au moins 6 résidus dont au moins 40% sont des résidus d'acide glutamique et/ou d'acide aspartique, dans lequel un variant d'une sous-unité a une séquence d'acides aminés qui présente au moins 70% d'identité avec la séquence de la sous-unité dont elle dérive et qui permet la formation d'un variant de C1q.

2. Procédé selon la revendication 1, dans lequel la séquence additionnelle d'acides aminés est située à l'extrémité C-terminale de l'une au moins des sous-unités ou de l'un au moins des variants de sous-unité.

3. Procédé selon l'une des revendications 1 ou 2, dans lequel la séquence additionnelle d'acides aminés est située à l'extrémité C-terminale de la sous-unité C1qC.

4. Procédé selon l'une des revendications 1 à 3, dans lequel la séquence additionnelle d'acides aminés est la suivante :
DYKDDDDK (SEQ ID NO : 8)

5. Procédé selon l'une des revendications 1 à 4, dans lequel les cellules expriment la sous-unité C1qA ou le variant de la sous-unité C1qA, la sous-unité C1qB ou le variant de la sous-unité C1qB, et la sous-unité C1qC, ou le variant de la sous-unité C1qC de manière stable.

6. Procédé selon l'une des revendications 1 à 5, dans lequel la protéine et C1q, ou le variant de la protéine C1q, est récupéré à partir du surnageant de la culture.

7. Protéine C1q, ou variant de la protéine Clq comprenant une sous-unité C1qA ou un variant de la sous-unité C1qA, une sous-unité C1qB ou un variant de la sous-unité C1qB, et une sous-unité C1qC, ou un variant de la sous-unité C1qC, et dont l'une au moins des sous-unités, ou l'un au moins des variants de sous-unité, porte en outre à l'extrémité N-terminale ou C-terminale une séquence additionnelle d'acides aminés d'au moins 6 résidus dont au moins 40% sont des résidus d'acide glutamique et/ou d'acide aspartique, dans lequel un variant d'une sous-unité a une séquence d'acides aminés qui présente au moins 70% d'identité avec la séquence de la sous-unité dont elle dérive et qui permet la formation d'un variant de C1q.

8. Protéine C1q ou variant de la protéine C1q selon la revendication 7, dans laquelle la séquence additionnelle d'acides aminés est telle que définie dans l'une des revendications 2 à 3.

9. Sous-unité ou variant de sous-unité de la protéine C1q portant à l'extrémité N-terminale ou C-terminale une séquence additionnelle d'acides aminés d'au moins 6 résidus dont au moins 40% sont des résidus d'acide glutamique et/ou d'acide aspartique,
dans laquelle un variant d'une sous-unité a une séquence d'acides aminés qui présente au moins 70% d'identité avec la séquence de la sous-unité dont elle dérive et qui permet la formation d'un variant de C1q, et dans laquelle la séquence additionnelle se situe à l'extrémité C-terminale de ladite sous-unité lorsque elle est portée par la sous-unité C1qA ou un variant de la sous-unité C1qA.

10. Sous-unité ou variant de sous-unité de la protéine C1q selon la revendication 9, dans laquelle la séquence additionnelle d'acides aminés est telle que définie dans l'une des revendications 2 à 3.

11. Acide nucléique codant pour une sous-unité ou variant de sous-unité de la protéine C1q telle que définie dans la revendication 9 ou 10.

12. Vecteur comprenant un acide nucléique tel que défini dans la revendication 11.

13. Cellule comprenant un acide nucléique tel que défini dans la revendication 11 ou un vecteur tel que défini dans la revendication 12.

14. Cellule selon la revendication 13, comprenant en outre un ou plusieurs acides nucléiques ou vecteurs codant pour les sous-unités ou variant de sous-unités complémentaires de C1q, de sorte que la cellule comprenne un ou plusieurs acides nucléiques ou vecteur codant pour une sous-unité C1qA ou un variant de la sous-unité C1qA, une sous-unité C1qB ou un variant de la sous-unité C1qB, et une sous-unité C1qC, ou un variant de la sous-unité C1qC, et dont l'une au moins des sous-unités, ou l'un au moins des variants de sous-unité, porte en outre à l'extrémité N-terminale ou C-terminale une séquence additionnelle d'acides aminés d'au moins 6 résidus dont au moins 40% sont des résidus d'acide glutamique et/ou d'acide aspartique.

15. Utilisation d'un acide nucléique selon la revendication 11 ou d'un vecteur selon la revendication 12, pour la transformation d'une cellule et la production de C1q par la cellule.

## Patentansprüche

1. Verfahren zur rekombinanten Herstellung eines Proteins C1q oder einer Variante des Proteins C1q, wobei das Protein ausgehend von einer *in-vitro-*Kultur von Zellen gewonnen wird, die eine Untereinheit C1qA oder eine Variante der Untereinheit C1qA, eine Untereinheit C1qB oder eine Variante der Untereinheit C1qB und eine Untereinheit C1qC oder eine Variante der Untereinheit C1qC exprimieren, und wobei mindestens eine der Untereinheiten oder mindestens eine der Untereinheitsvarianten ferner am N-terminalen oder C-terminalen Ende eine zusätzliche Aminosäuresequenz von mindestens 6 Resten trägt, von denen mindestens 40 % Glutaminsäure- und/oder Asparaginsäurereste sind, wobei eine Variante einer Untereinheit eine Aminosäuresequenz aufweist, die mindestens 70 % Identität mit der Sequenz der Untereinheit, von der sie abgeleitet ist, aufweist und die die Bildung einer Variante von C1q ermöglicht.

2. Verfahren nach Anspruch 1, wobei sich die zusätzliche Aminosäuresequenz am C-terminalen Ende von mindestens einer der Untereinheiten oder mindestens einer der Untereinheitsvarianten befindet.

3. Verfahren nach einem der Ansprüche 1 oder 2, wobei sich die zusätzliche Aminosäuresequenz am C-terminalen Ende der Untereinheit C1qC befindet.

4. Verfahren nach einem der Ansprüche 1 bis 3, wobei die zusätzliche Aminosäuresequenz die folgende ist:
DYKDDDDK (SEQ ID NR: 8)

5. Verfahren nach einem der Ansprüche 1 bis 4, wobei die Zellen die Untereinheit C1qA oder die Variante der Untereinheit C1qA, die Untereinheit C1qB oder die Variante der Untereinheit C1qB und die Untereinheit C1qC oder die Variante der Untereinheit C1qC stabil exprimieren.

6. Verfahren nach einem der Ansprüche 1 bis 5, wobei das Protein und C1q oder die Variante des Proteins C1q ausgehend von dem Überstand der Kultur gewonnen wird.

7. Protein C1q oder Variante des Proteins C1q, umfassend eine Untereinheit C1qA oder eine Variante der Untereinheit C1qA, eine Untereinheit C1qB oder eine Variante der Untereinheit C1qB und eine Untereinheit C1qC oder eine Variante der Untereinheit C1qC, und wobei mindestens eine der Untereinheiten oder mindestens eine der Untereinheitsvarianten ferner am N-terminalen oder C-terminalen Ende eine zusätzliche Aminosäuresequenz von mindestens 6 Resten trägt, von denen mindestens 40 % Glutaminsäure- und/oder Asparaginsäurereste sind, wobei eine Variante einer Untereinheit eine Aminosäuresequenz aufweist, die mindestens 70 % Identität mit der Sequenz der Untereinheit, von der sie abgeleitet ist, aufweist und die die Bildung einer Variante von C1q ermöglicht.

8. Protein C1q oder Variante des Proteins C1q nach Anspruch 7, wobei die zusätzliche Aminosäuresequenz wie in einem der Ansprüche 2 bis 3 definiert ist.

9. Untereinheit oder Untereinheitsvariante des Proteins C1q, die am N-terminalen oder C-terminalen Ende eine zusätzliche Aminosäuresequenz von mindestens 6 Resten trägt, von denen mindestens 40 % Glutaminsäure- und/oder Asparaginsäurereste sind, wobei eine Variante einer Untereinheit eine Aminosäuresequenz aufweist, die mindestens 70 % Identität mit der Sequenz der Untereinheit, von der sie abgeleitet ist, aufweist und die die Bildung einer Variante von C1q ermöglicht, und wobei die zusätzliche Sequenz sich am C-terminalen Ende der Untereinheit befindet, wenn sie von der Untereinheit C1qA oder einer Variante der Untereinheit C1qA getragen wird.

10. Untereinheit oder Untereinheitsvariante des Proteins C1q nach Anspruch 9, wobei die zusätzliche Aminosäuresequenz wie in einem der Ansprüche 2 bis 3 definiert ist.

11. Nukleinsäure, die für eine Untereinheit oder Untereinheitsvariante des Proteins C1q wie in Anspruch 9 oder 10 definiert kodiert.

12. Vektor, umfassend eine Nukleinsäure wie in Anspruch 11 definiert.

13. Zelle, umfassend eine Nukleinsäure wie in Anspruch 11 definiert oder einen Vektor wie in Anspruch 12 definiert.

14. Zelle nach Anspruch 13, ferner umfassend eine oder mehrere Nukleinsäuren oder Vektoren, die für die Untereinheiten oder Untereinheitenvariante kodieren, die komplementär zu C1q sind, wobei die Zelle eine oder mehrere Nukleinsäuren oder einen Vektor umfasst, der für eine Untereinheit C1qA oder eine Variante der Untereinheit C1qA, eine Untereinheit C1qB oder eine Variante der Untereinheit C1qB und eine Untereinheit C1qC oder eine Variante der Untereinheit C1qC kodiert, und wobei mindestens eine der Untereinheiten oder mindestens eine der Untereinheitsvarianten ferner am N-terminalen oder C-terminalen Ende eine zusätzliche Aminosäuresequenz von mindestens 6 Resten trägt, von denen mindestens 40 % Glutaminsäure- und/oder Asparaginsäurereste sind.

15. Verwendung einer Nukleinsäure nach Anspruch 11 oder eines Vektors nach Anspruch 12 für die Transformation einer Zelle und die Produktion von C1q durch die Zelle.

## Claims

1. Method for producing a protein C1q, or a variant of the protein C1q, by recombinant means, wherein the protein is recovered from an *in vitro* culture of cells expressing a subunit C1qA or a variant of the subunit C1qA, a subunit C1qB or a variant of the subunit C1qB, and a subunit C1qC, or a variant of the subunit C1qC, and of which at least one of the subunits, or at least one of the subunit variants, furthermore carries at the N-terminal or C-terminal end, an additional sequence of amino acids of at least 6 residues of which at least 40 % are glutamic acid and/or aspartic acid residues, wherein a variant of a subunit has a sequence of amino acids which presents at least 70 % identity with the sequence of the subunit, from which it derives and which makes it possible for the formation of a C1q variant.

2. Method according to claim 1, wherein the additional sequence of amino acids is situated at the C-terminal end of at least one of the subunits or at least one of the subunit variants.

3. Method according to one of claims 1 or 2, wherein the additional sequence of amino acids is situated at the C-terminal end of the subunit C1qC.

4. Method according to one of claims 1 to 3, wherein the additional sequence of amino acids is as follows:
DYKDDDDK (SEQ ID NO: 8)

5. Method according to one of claims 1 to 4, wherein the cells express the subunit C1qA or the variant of the subunit C1qA, the subunit C1qB or the variant of the subunit C1qB, and the subunit C1qC, or the variant of the subunit C1qC in a stable manner.

6. Method according to one of claims 1 to 5, wherein the protein and C1q, or the variant of the protein C1q, is recovered from the supernatant of the culture.

7. Protein C1q, or variant of the protein C1q comprising a subunit C1qA or a variant of the subunit C1qA, a subunit C1qB or a variant of the subunit C1qB, and a subunit C1qC, or a variant of the subunit C1qC, and of which at least one of the subunits, or at least one of the subunit variants, furthermore carries at the N-terminal or C-terminal end, an additional sequence of amino acids of at least 6 residues of which at least 40 % are glutamic acid and/or aspartic acid residues, wherein a variant of a subunit has a sequence of amino acids which presents at least 70 % identity with the sequence of the subunit from which it derives and which makes it possible for the formation of a C1q variant.

8. Protein C1q or variant of the protein C1q according to claim 7, wherein the additional sequence of amino acids is such as defined in one of claims 2 to 3.

9. Subunit or subunit variant of the protein C1q carrying at the N-terminal or C-terminal end, an additional sequence of amino acids of at least 6 residues, of which at least 40 % are glutamic acid and/or aspartic acid residues, wherein a variant of a subunit has a sequence of amino acids which presents at least 70 % identity with the sequence of the subunit from which it derives and which makes it possible for the formation of a C1q variant and wherein the additional sequence is situated at the C-terminal end of said subunit when it is carried by the subunit C1qA or a variant of the subunit C1qA.

10. Subunit or subunit variant of the protein C1q according to claim 9, wherein the additional sequence of amino acids is such as defined in one of claims 2 to 3.

11. Nucleic acid encoding a subunit or subunit variant of the protein C1q such as defined in claim 9 or 10.

12. Vector comprising a nucleic acid such as defined in claim 11.

13. Cell comprising a nucleic acid such as defined in claim 11 or a vector such as defined in claim 12.

14. Cell according to claim 13, further comprising one or more nucleic acids or coding vectors for subunits or complementary C1q subunit variants, such that the cell comprises one or more nucleic acids or coding vectors for a subunit C1qA or a variant of the subunit C1qA, a subunit C1qB or a variant of the subunit C1qB, and a subunit C1qC, or a variant of the subunit C1qC, and of which at least one of the subunits, or at least one of the subunit variants, furthermore carries at the N-terminal or C-terminal end an additional sequence of amino acids of at least 6 residues, of which at least 40 % are glutamic acid and/or aspartic acid residues.

15. Use of a nucleic acid according to claim 11 or of a vector according to claim 12, for the transformation of a cell and the production of C1q by the cell.
